# EUROPEAN PATENT SPECIFICATION

(11) **EP 4 025 138 B1**
(45) Date of publication and mention of the grant of the patent: **17.07.2024**
(21) Application number: 20764123.4
(22) Date of filing: 03.09.2020
(51) Int. Cl.: A61B 17/17, A61B 17/74, A61B 17/92

(54) **SURGICAL SYSTEM FOR OSTEOSYNTHESIS OF FEMORAL FRACTURES**
CHIRURGISCHES SYSTEM ZUR OSTEOSYNTHESE VON FEMURFRAKTUREN
SYSTÈME CHIRURGICAL POUR L'OSTÉOSYNTHÈSE DE FRACTURES FÉMORALES

(30) Priority: 03.09.2019 EP 19195177
(43) Date of publication of application: 13.07.2022
(73) Proprietor: Bispebjerg Hospital, 2400 Copenhagen NV (DK)
(72) Inventor: CHRISTENSEN, Mikkel, Holm, 2400 Copenhagen NV (DK); PALM, Henrik, 2400 Copenhagen NV (DK); LAURITZEN, Jes, Bruun, 2400 Copenhagen NV (DK)
(74) Representative: Aera A/S
(86) International application number: PCT/EP2020/074607
(87) International publication number: WO 2021/043907

(56) References cited:
- EP-A1- 0 976 365
- EP-A2- 1 839 607
- EP-A2- 1 867 294
- DE-U1- 202004 014 226
- FR-A1- 2 647 006
- US-A- 5 352 228
- US-A- 6 120 504
- US-A1- 2008 140 077
- US-A1- 2018 116 747

## Description

The present disclosure relates to a surgical system for osteosynthesis of femoral fractures, individual parts of such system.

### BACKGROUND

Femoral fractures are typically treated by internal fixation using an intramedullary (IM) nail, possibly in mechanical connection with a hip screw in case of intertrochanteric or subtrochanteric fractures, see e.g. US 2008/0140077A1, US 6,221,074 or US 6,562,042.

Obese patients with femoral fractures are difficult to osteosynthesize with IM nail and hip screw. The obesity makes the antegrade insertion of the intramedullary nail technical demanding or even impossible to insert from the usual entry point proximal to the greater trochanter of femur. The surplus amounts of fatty tissue block access to the point of entry for the surgical instruments, both when preparing the medullary canal (reamer, cutter, drill) and when inserting the IM nail (jig, IM nail and hammer) - all of which should be done under the correct angle. The obesity makes the insertion of the intramedullary nail technical demanding and even impossible to insert from the usual entry point proximal to the greater trochanter of the femur. The surgery requires a very extensive surgical approach to insert the conventional intramedullary nail with subsequent hip sliding screw. The consequences for these patients may be at worst severe surgical complications with wound leakage, deep infections and even fatal outcome, which is not uncommon. Complication rate is seriously high in this patient category. There is therefore a desire to reduce the risk for patients and the surgical complexity of the procedure.

US 6,221,074 (also published as EP 1867294A2) describes a number of osteosyntheses with both antegrade and retrograde insertion of the IM nail. Retrograde nailing, where the IM nail is introduced via the knee joint is a known alternative that works well for obese patients. This retrograde approach is however not applicable for trochanteric and subtrochanteric fractures since no solutions for a mechanical connection with a hip screw exists.

### SUMMARY

Accordingly, there is a need for a surgical system for osteosynthesis of femoral fractures with ease of access on all patients.

An orthopaedic implant for osteosynthesis of femoral fractures is disclosed, comprising:
- an intramedullary nail for retrograde insertion in the femur, the intramedullary nail comprising a leading end part to be positioned proximally in the femur and a trailing end part to be positioned distally in the femur;
- a femoral neck screw elongated along a major axis and an at least partial, external thread running along the major axis in a first end part of the femoral neck screw for fastening the first end part subchondrally in a femoral head by a screwing action, the femoral neck screw comprising a through-hole configured to receive the leading end part of the intramedullary nail along a first direction having an angle between 110° and 150° to the major axis;
- wherein the intramedullary nail comprises a stop for engaging the femoral neck screw to prevent the leading end part from extending so far through the through-hole as to reach the cortex of the greater trochanter.

In the following, a number of preferred and/or optional features, elements, examples and implementations will be summarized. Features or elements described in relation to one embodiment or disclosure may be combined with or applied to the other embodiments or aspects where applicable. For example, structural and functional features applied in relation to a method may also be used as features in relation to a device or system and vice versa.

The intramedullary nail is designed for retrograde osteosynthesis of femoral fractures in that its leading end part is configured to - by means of its shape, dimensions, and/or the stop - form a mechanical connection with the femoral neck screw.

The section of the leading end part of the IM nail that extends through the through-hole will typically not contribute to the stability provided by the osteosynthesis, and it should not continue so far as to reach and potentially damage the greater trochanter. As will be described later, there are embodiments where it is advantageous when the leading part can extend somewhat through the through-hole. Hence, in other embodiments, the intramedullary nail comprises a stop for engaging the femoral neck screw to prevent the leading end part from extending more than 3 centimeters through the through-hole, such as more than 2 centimeters through the through-hole or more than 1 centimeter through the through-hole.

In further embodiments, the stop of the intramedullary nail involves a tapering with a smaller cross section nearer its leading end part. Similarly, the through-hole of the neck screw has a tapering with a larger cross section on a side for accepting/receiving the leading end part. The taperings are complementary and positioned relative to the leading end part to stop the intramedullary nail from extending so far through the through-hole as to reach the cortex of the greater trochanter (or from extending more than 3 centimeters through the through-hole, such as more than 2 centimeters or more than 1 centimeter). The taperings are also complementary to laterally fixate the intramedullary nail relative to the femoral neck screw when the taperings engage.

In further embodiments, the leading end part of the intramedullary nail and the femoral neck screw involves structural features for preventing rotation of the intramedullary nail around the first direction and/or laterally fixating the intramedullary nail relative to the femoral neck screw after the leading end part has been received by the through-hole. These structural features may include the stop for engaging the femoral neck screw to prevent the leading end part from extending too far through the through-hole as described above.

In further embodiments, the neck screw is cannulated along at least part of the major axis and comprises, in a second end part opposite the first end part, an internal thread along the cannulation, further comprising a set screw having an external thread corresponding to the internal thread along the cannulation and having a primary end part adapted to engage the leading end part of the intramedullary nail when the leading end part is received by the through-hole and the set screw is screwed towards the first end part of the femoral neck screw. The engaging between the primary end part of the set screw and the leading end part of the intramedullary nail may provide one or more of the following functions: preventing the leading end part from extending too far through the through-hole as described above, laterally fixating the intramedullary nail relative to the femoral neck screw, preventing rotation of the intramedullary nail around the first direction when the leading end part is received by the through-hole.

In a further embodiment, the leading end part of the intramedullary nail comprises a recess or a hole for receiving the primary end part of the set screw to prevent rotation of the intramedullary nail around the first direction when the leading end part is received by the through-hole and the set screw is screwed towards the first end part of the femoral neck screw.

In a further embodiment, at least a section of the leading end part of the intramedullary nail may have a larger cross-sectional diameter than a middle part of the IM nail. Narrower or more pointy ends have a tendency to get wedged during insertion in the femoral canal (which is curved in both the frontal and the axial plane). Am IM nail with a bulkier leading end part and a thinner, more flexible middle part will provide the advantage of making the insertion easier and reduce the risk of wedging or jamming. In a further embodiment, the cross section of the middle part of the IM nail may have a flat aspect ratio, such as larger than 1,5:1, 2:1, or 2,5:1. The aim is to secure flexibility of the nail during insertion in the curved femoral canal while adding strength to the implant and avoid breakage.

The orthopaedic implant disclosed is preferably adapted for use in a method of osteosynthesis of femoral fractures comprising
- retrogradely inserting an intramedullary nail in a femur of a subject, and pausing the insertion when a leading end part of the intramedullary nail reaches the area just short of where a femoral neck screw is to be placed (e.g. 2 cm short of the extended line from the center of caput femoris and parallel to the collum axis in the AP plane)
- inserting a femoral neck screw into the femoral neck and fixing it in the femoral head, the femoral neck screw comprising a through-hole configured to receive the leading end part of the intramedullary nail along a first direction having an angle between 110° and 150° to a major axis of the femoral neck screw, comprising aligning the through-hole with the leading end part of the intramedullary nail;
- continuing the retrograde insertion of the intramedullary nail so that the leading end part is received by the through-hole of the femoral neck screw.

The method is preferably used in osteosynthesis of intertrochanteric or subtrochanteric femoral fractures where a combination of an intramedullary nail and a femoral neck screw is desired.

The orthopaedic implant disclosed herein is preferably adapted for use in the disclosed method. In this respect, several features of the orthopaedic implant are advantageous individually and in combination, including:
- The intramedullary nail is adapted to be inserted retrograde.
- The femoral neck screw has a through-hole for receiving the intramedullary nail. This is opposite to the configuration commonly used in the prior art where the IM nail has a through hole for receiving the neck screw, see e.g. US 7,455,673. Such prior art configurations (through-hole in IM nail) are, however, configured for antegrade insertion of the IM nail since the section of the IM nail having the through-hole must be thick enough for receiving the neck screw and therefore has to be formed in the trailing end part. Having such thick section in a leading end part of the IM, as would be required for retrograde insertion, would not be possible.
- Configurations where the femoral neck screw has a through-hole for receiving the IM nail are known from e.g. US 6,221,074 or EP 1867294A2. With retrograde insertion of the IM nail, it would have to be the leading part of the IM nail that formed a mechanical connection with the femoral neck screw, not the trailing part. The prior art configurations (of through-hole in neck screw) are, however, configured solely for antegrade insertion of the IM nail.
- The leading end part of the intramedullary nail has a stop so that only the leading end part can be received by the through-hole of the neck screw - a middle part or trailing end part of the IM nail cannot enter the through-hole. As will be described in greater detail later, this prevents inserting the IM nail too far as well as sliding of the neck screw on the IM nail.

A system for osteosynthesis of femoral fractures is disclosed. The system comprises an orthopaedic implant as previously disclosed herein and a jig for inserting the intramedullary nail and aligning the femoral neck screw relative to the intramedullary nail, the jig being configured to form a rigid mechanical connection to a trailing end part of the intramedullary nail so that the leading end part of the intramedullary nail is held along a first direction, the jig comprising a first guide for drilling for the femoral neck screw, the first guide defining an aiming line intersecting the first direction; wherein the intersection between the aiming line and the first direction lies beyond the extension of the intramedullary nail, when the intramedullary nail is connected to the jig.

The disclosed system is preferably adapted for use in the disclosed method. In this respect, several features of the system - together with the features of the orthopaedic implant listed above - are advantageous individually and in combination, including:
- the intersection between the aiming line and the first direction lies beyond the extension of the intramedullary nail, when the intramedullary nail is connected to the jig. In prior art configurations, this intersection would typically lie in the trailing end part of the IM nail, either because the neck screw is configured to go through the IM nail (through-hole in IM nail) or because the nail is not held by the jig during insertion of the neck screw (through-hole in neck screw).

The length of the femur varies with patient age and size, and the length of the IM nail should be selected to correspond to the length of the femur. Therefore, the system is preferably configured to use IM nails of different lengths. In further embodiments, the system comprises a set of instructions comprising instructions for a user to use a combination of a guide and intramedullary nail length for which an intersection between the aiming line and the first direction lies beyond the extension of the intramedullary nail, when the intramedullary nail is connected to the jig.

In further embodiments, the jig comprises at least a second guide for drilling for the femoral neck screw and defining an aiming line, a position of the second guide being a translation of the position of the first guide along a line at least substantially parallel to the first direction, the system further comprising a set of instructions comprising instructions for a user to, for a given a length of the intramedullary nail, use the one of the first and second guides that results in a distance between the intersection and the intramedullary nail being closer to, but larger than, a radius of the femoral neck screw at the position of the through-hole.

In further embodiments, a length of the jig below a position of the first guide can be adjusted so that the intersection between the aiming line and the first direction can be moved along the first direction, further comprising a set of instructions comprising instructions for a user to, for a given a length of the intramedullary nail to be connected to the jig, adjust the length of the jig so that a distance between the intersection and the intramedullary nail is as small as possible while being larger than a radius of the femoral neck screw at the through-hole.

In further embodiments the jig has a J-shape with a short end part, a curved middle part, and a long end part, the short end part being configured to form a rigid mechanical connection to a trailing end part of an intramedullary nail so that the intramedullary nail is held along a first direction at least substantially parallel to the long end part, the long end part comprising a first guide for drilling for the femoral neck screw, the first guide defining an aiming line intersecting the first direction; wherein a length of the long end part can be adjusted so that an intersection between the aiming line and the first direction can be moved along the first direction.

In further embodiments, the long end part of the jig comprises a scale indicating the length to which the long end part should be adjusted for an intramedullary nail of a given length held at the short end part. Preferably, the scale is calibrated so that the intersection between the aiming line and the first direction lies beyond the extension of an intramedullary nail with the given length, when the intramedullary nail is held at the short end part.

The present disclosures allow for improved osteosynthesis of femoral fractures, in particular intertrochanteric or subtrochanteric femoral fractures where a combination of an intramedullary nail and a femoral neck screw is desired, but where antegrade insertion of the IM nail is difficult or impossible. The disclosed orthopaedic implant, IM nail, system and jig are all adapted for use in the disclosed surgical procedure which combine two different osteosynthesis techniques: the intramedullary hip screw for hip fractures and the retrograde femoral nail for femoral shaft fractures. These techniques were previously not combined since the antegrade insertion of the IM nail is standard when a combination with femoral neck screw is required.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
Fig. 1 schematically illustrates an exemplary orthopaedic implant and an intramedullary nail according to the disclosure.
Figs. 2-4 schematically illustrate different embodiments of an orthopaedic implant and an intramedullary nail according to the disclosure.
Fig. 5A-E illustrate exemplary application of the orthopaedic implant according to the disclosure.
Figs. 6-7 illustrate exemplary jigs according to the disclosure.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

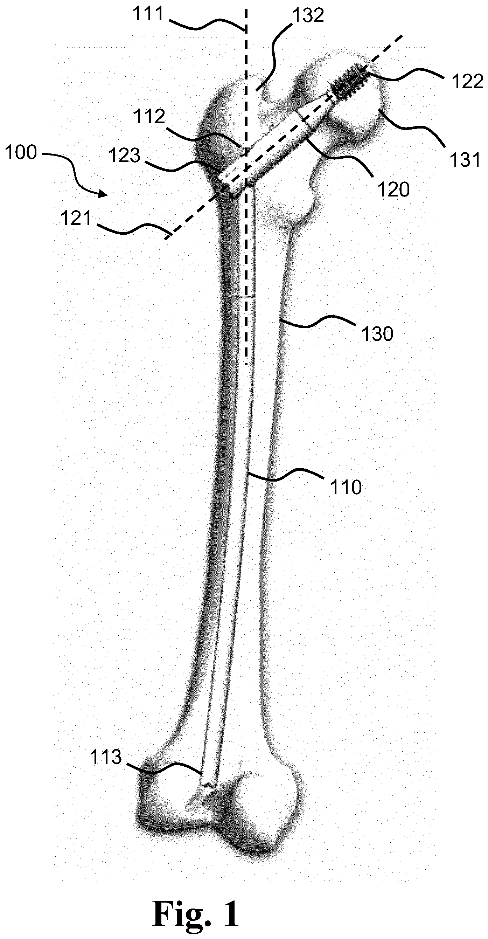

Fig. 1 shows an assembled exemplary orthopaedic implant 100 involving an intramedullary nail 110 overlaid on an illustration of a femur 130. The intramedullary nail 110 comprises a leading end part 112 to be positioned proximally in the femur 130 and extending along a first direction 111. The intramedullary nail 110 also comprises a trailing end part 113 to be positioned distally in the femur 130. The orthopaedic implant 100 comprises a femoral neck screw 120 elongated along a major axis 121 and having a first end part 122 to be fastened subchondrally in a femoral head 131 of the femur 130 and a second end part 123 opposite the first end part 122.

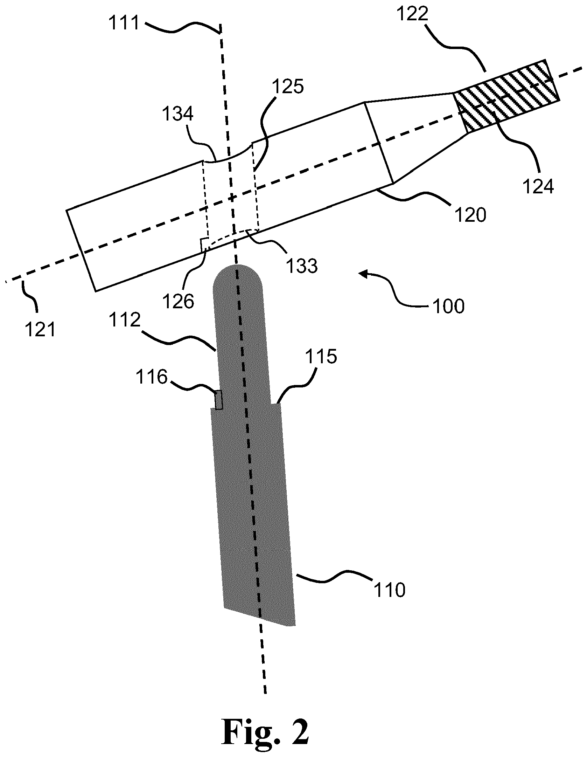

Fig. 2 shows a disassembled exemplary orthopaedic implant 100 involving an intramedullary nail 110. The femoral neck screw 120 is elongated along the major axis 121 and an at least partial, external thread 124 is running along the major axis in the first end part 122 of the femoral neck screw for fastening the first end part subchondrally in a femoral head by a screwing action. The femoral neck screw 120 comprises a through-hole 125 configured to receive the leading end part 112 of the intramedullary nail 110 along the first direction 111 having an angle between 110° and 150° to the major axis 121. The through-hole 125 has a distal rim 133 and a proximal rim 134. The intramedullary nail 110 comprises a stop 115 for engaging the femoral neck screw 120 to prevent the leading end part 112 from extending so far through the through-hole 125 as to reach the cortex of the greater trochanter 132 (see Fig. 1). In the embodiment shown in Fig. 2, the stop 115 is an increased diameter of the IM nail 110 that is larger than the diameter of the through-hole 125. In a further embodiment, the stop 115 comprises a protrusion 116 and the neck screw 120 comprises, at the edge of the through-hole 125, a notch 126 with a shape and size corresponding to the protrusion 116. When the stop 115 engages the neck screw 120, the protrusion 116 will be inserted in the notch 126 and prevent rotation of the intramedullary nail 110 around the first direction 111.

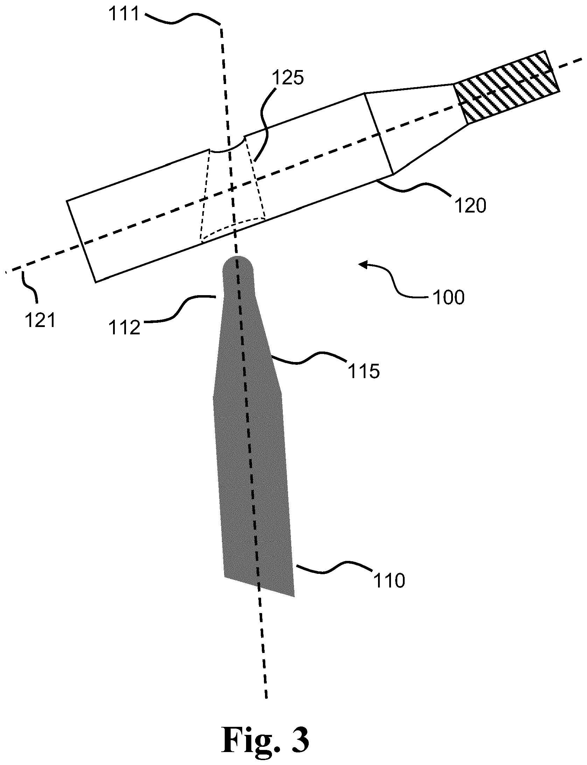

Fig. 3 shows a disassembled exemplary orthopaedic implant 100 involving an intramedullary nail 110. In the embodiment shown in Fig. 3, the stop 115 involves a tapering with a smaller cross section nearer the leading end part 112 and the through-hole 125 has a tapering with a larger cross section on a side for receiving the leading end part 112. The taperings of the IM nail 110 and the through-hole 125 are complementary, i.e. have the same or similar angles and sizes, so that when the leading end part 112 is received by the through-hole 25, the surfaces of the taperings will engage to stop the intramedullary nail 110 from extending further into or through the through-hole 125. This engagement will further laterally fixate the intramedullary nail 110 relative to the femoral neck screw 120. When the surfaces of the taperings are engaged, the friction forces may also prevent rotation of the intramedullary nail 110 around the first direction 111.

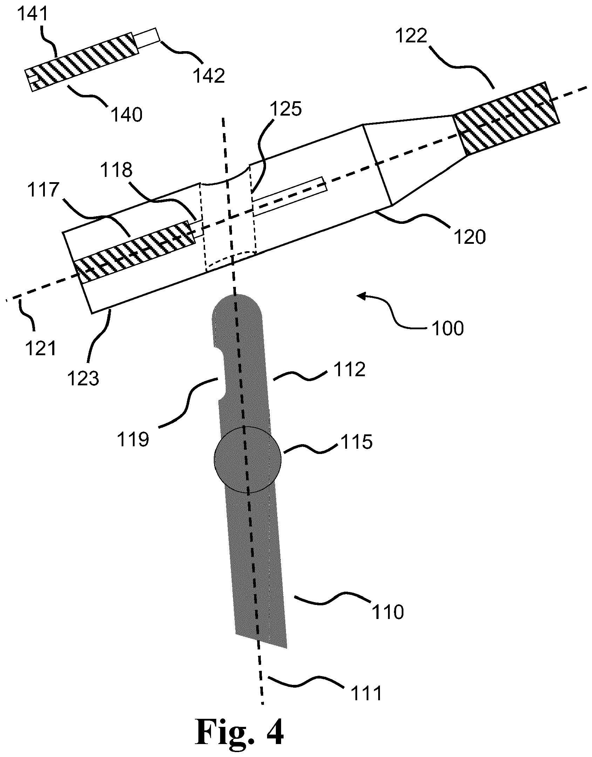

Fig. 4 shows a disassembled exemplary orthopaedic implant 100 involving an intramedullary nail 110. Fig. 4 illustrates an embodiment where the stop 115 involves a member, preferably sphere-shaped, with a cross-sectional diameter larger than the through-hole 125. In one embodiment, a position of the stop along the first direction 111 may be adjusted in order to adjust the effective length of the IM nail, i.e. the distance from the trailing end part 113 to the femoral neck 120 in the assembled implant 100 - please refer to Fig. 1. Increasing the effective length of the IM nail 110 by moving the stop 115 away from the leading end part 112 would result in the leading end part 112 extending further through the through-hole 125 when the implant is assembled. As discussed previously, the section of the leading end part 112 of the IM nail that extends through the through-hole 125 will typically not contribute to the stability provided by the osteosynthesis. It should however not extend so far as to reach and potentially damage the greater trochanter 132 (see Fig. 1). However, in adult patients there are typically at least 3 centimeters of margin between the neck screw 120 and the greater trochanter 132. This means that the effective length of the IM nail 110 can be adjusted up to 3 centimeters. This has the advantage that the same IM nail can be used for patients with different size femurs and thus reduce the stock of IM nails of different length.

There are other practical considerations regarding the extension of the leading end part 112 through the through-hole 125. During insertion, the surgeon monitors the position of the nail while hammering on the jig. The events when the leading end part 112 enters the distal rim 133 of the through-hole 125, when it exits the proximal rim 134 of the through-hole 125, and when the stop 115 engages the femoral neck screw are of particular interest. If the stop 115 is positioned too close to a tip of the leading end part, the leading end part will not exit the proximal rim 134 of the through-hole 125 before the stop engages the femoral neck screw. While the section of the leading end part 112 that has exited the proximal rim 134 of the through-hole 125 is typically not important for the stability of the osteosynthesis, it is advantageous if it extends at least somewhat beyond the proximal rim. Firstly, the section of the leading end part 112 that engages the proximal rim 134 does contribute to the stability, in particular to forces working on the angle between the first direction 111 and the major axis 121. Therefore, in one embodiment, the leading end part of the intramedullary nail is configured to, by means of its shape and the position of the stop, engage the proximal rim of the through-hole when the stop engages the femoral neck screw. This means that the leading end part touches the femoral neck screw along the proximal rim which provides the advantage of increased mechanical stability as described above. If, for example, the leading end part that extends through the proximal rim when the stop engages the femoral neck screw is tapered, it would not engage the proximal rim. Secondly, the surgeon only has a 2D projection of the osteosynthesis and the femur as guidance. The tip exiting the proximal rim 134 of the through-hole 125 is therefore an important visual clue for the surgeon, and the increment in the distance between the tip and the femoral neck screw for each hammer stroke may be the most precise indication of the progress. Naturally, one wants to progress slowly when the stop is about to engage the femoral neck screw, and not have them slamming together with a powerful hammer-stroke since that would increase the risk of jamming and damage to the parts. Therefore, in one embodiment, the stop is positioned to allow a tip of the leading end part to extend at least 3 mm, such as at least 5 mm or at least 1 cm beyond the proximal rim 134 of the through-hole when the stop engages the femoral neck screw. This provides the advantage of visual guiding to the surgeon as described above. As previously described in relation to another embodiment, the stop is preferably also positioned to prevent the leading end part from extending more than 3 centimeters such as more than 2 centimeters through the through-hole or more than 1 centimeter through the through-hole, when the stop engages the femoral neck screw. These embodiments are preferably combined to provide the shape and position of the stop.

Fig. 4 also illustrates an embodiment where the neck screw 120 is cannulated along at least part of the major axis 121 and comprises, in the second end part 123, an internal thread 117 along the cannulation 118. In the embodiment, the implant 100 further comprises a set screw 140 having an external thread 141 corresponding to the internal thread 117 along the cannulation 118 and having a primary end part 142 adapted to engage the leading end part 112 of the intramedullary nail when the leading end part is received by the through-hole and the set screw is screwed towards the first end part of the femoral neck screw. In a further embodiment, also illustrated in Fig. 4, the leading end part 112 comprises a recess 119 (could also be a hole, not shown) for receiving the primary end part 142 of the set screw 140 to prevent rotation of the intramedullary nail 110 around the first direction 111 when the leading end part 112 is received by the through-hole 125 and the set screw 140 is screwed towards the first end part 122 of the femoral neck screw 120.

The different stops 115, the protrusion 116 and the notch 126, the set screw 140, and the recess 119 described in relation to Figs. 2-4 represent different structural features preventing rotation of the intramedullary nail 110 around the first direction 111 after the leading end part 112 has been received by the through-hole 125.

At least a section of the leading end part 112 of the IM nail may have a larger cross-sectional diameter than a middle part of the IM nail in order to make the insertion easier and reduce the risk of wedging or jamming. Preferably, a cross section of the middle part of the IM nail may even have a flat aspect ratio, such as larger than 1,5:1, 2:1, or 2,5:1 in order to increase flexibility of the IM nail during insertion in the curved femoral canal. The trailing end part 113 preferably then has a larger cross-sectional diameter than the middle part to allow for the mechanical coupling to the jig and to absorb and distribute the impact of the hammering on the jig during insertion.

Orthopaedic implants for osteosynthesis of femoral fractures are well known in the field. The implants of the present disclosures are generally based on similar implants available in the prior art when it comes to materials, shapes and dimensions of parts that are not influenced by the disclosed features. The person skilled in the art of orthopaedic implant design will therefore be able to design and produce implants according to the present disclosures based on the descriptions provided herein.

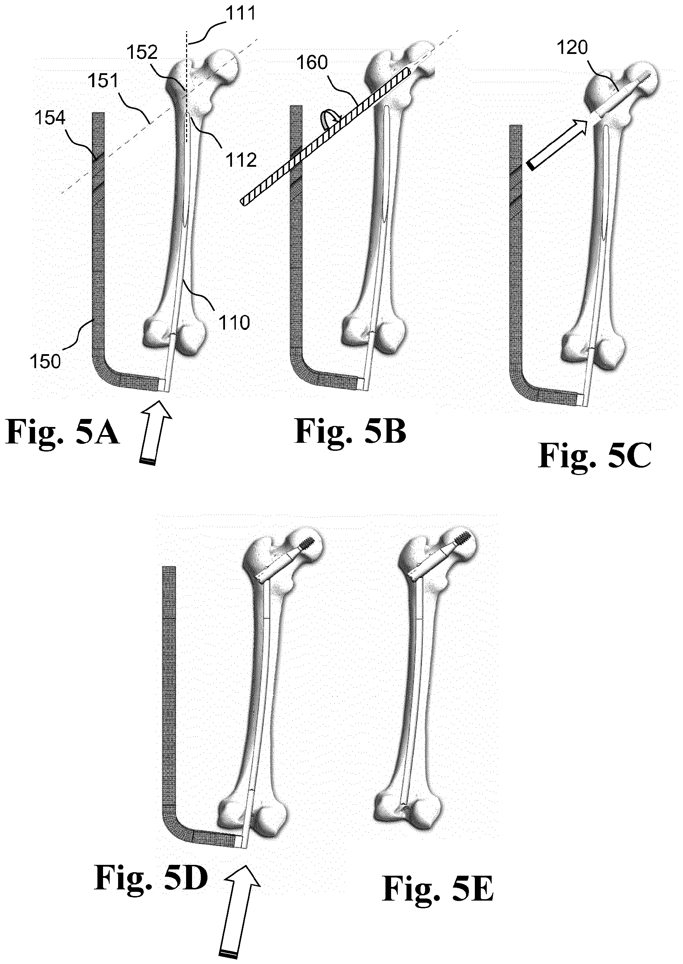

Figs. 5A-E illustrates an exemplary application of the orthopaedic implant. Fig. 5A shows retrogradely inserting an intramedullary nail 110 through the femoral canal of a subject from the notch between the femoral condyles at the knee joint level. The insertion is paused when a leading end part 112 of the intramedullary nail reaches the area just short of where a femoral neck screw is to be placed. In a preferred embodiment, this will be between 1-3 cm short of the extended line from the center of caput femoris and parallel to the collum axis in the anteroposterior (AP) plane. Fig. 5C shows inserting a femoral neck screw 120 into the femoral neck and fixing it in the femoral head, the femoral neck screw comprising a through-hole configured to receive the leading end part 112 of the intramedullary nail along a first direction having an angle between 110° and 150° to a major axis of the femoral neck screw, comprising aligning the through-hole with the leading end part 112 of the intramedullary nail. Fig. 5D shows continuing the retrograde insertion of the intramedullary nail 110 so that the leading end part is received by the through-hole of the femoral neck screw 120..

Naturally, a complete surgical procedure involves many more steps which are known from the prior art. For example, drilling out the femoral canal prior to insertion of the IM nail (not shown), stabilizing the nail-jig construct using a drill through a drill hole in the IM nail closer to the fracture site (not shown), drilling for the femoral neck screw using a first guide for drilling in a jig (shown in Fig. 5B), distally locking the IM nail through the jig after passing it through the through-hole of the neck screw (not shown). Fig. 5E illustrates the inserted implant after removal of the jig.

After completion of the osteosynthesis and a prescribed healing period, the patient may stand up, resulting in a load on the femoral neck. At this stage, the bone may or may not have grown together completely, and load on the femoral neck may result in a downward force on the femoral neck screw. The stop comprised by the disclosed orthopaedic implant and IM nail prevents the femoral neck screw from sliding downwards on the IM nail. Prior art configurations with through-hole in neck screw such as US 6,221,074 use a set screw inside the neck screw that goes through the nail to stop such sliding of the neck screw. A set screw only pressing on the nail could not prevent this sliding. Since the disclosed IM nail "comes from below", the stop provides a simple and strong solution preventing the IM nail from being pushed further up through the through-hole of the neck screw.

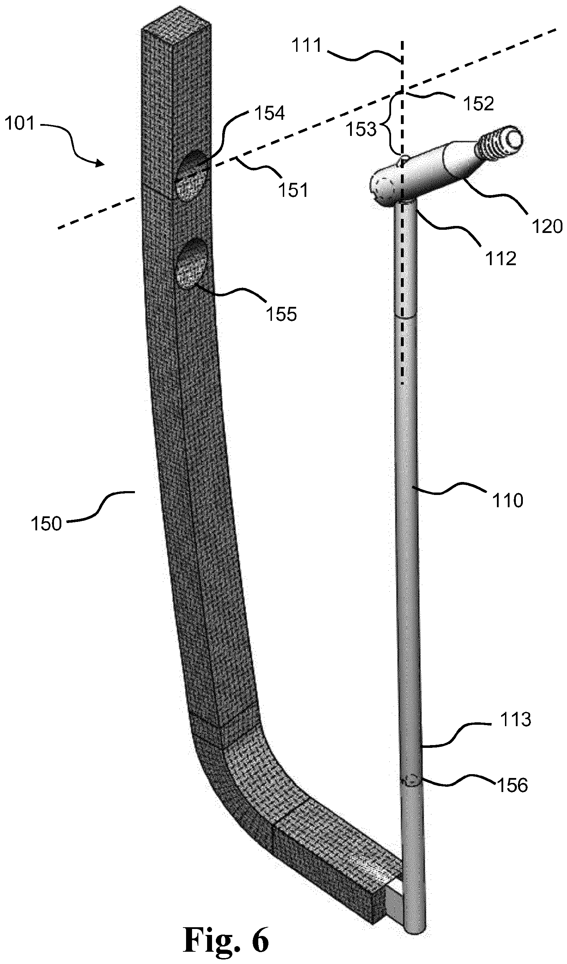

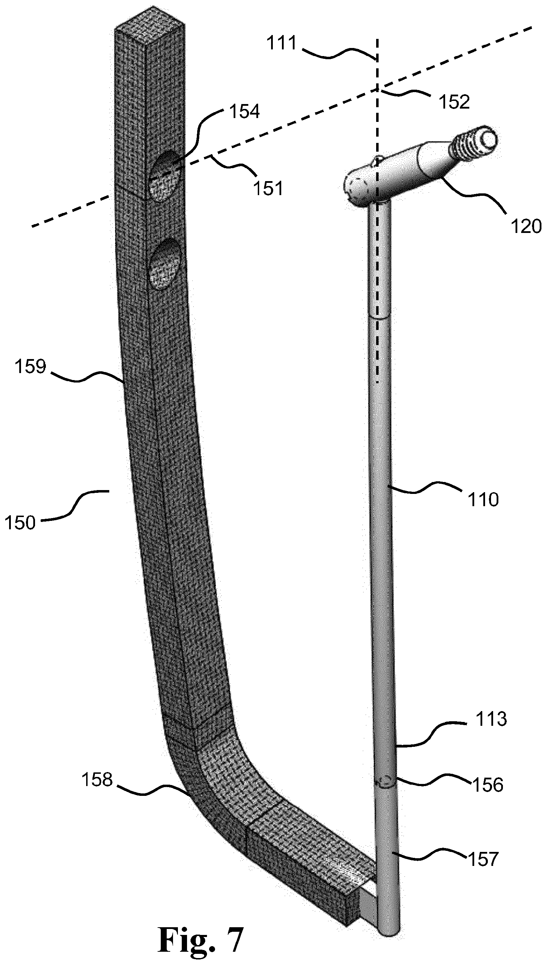

Fig. 6 illustrates a system 101 for osteosynthesis of femoral fractures comprising an orthopaedic implant with an IM nail 110 and a femoral neck screw 120 as described previously, and a jig 150 for inserting the intramedullary nail 110 and aligning the femoral neck screw 120 relative to the intramedullary nail. In Figs. 6 and 7, the femoral neck screw 120 is attached to the IM nail for purposes of illustration only - the jig with the IM nail are not assembled with the neck screw until the step illustrated in Fig. 5C.

The jig 150 is configured to form a rigid mechanical connection 156 to the trailing end part 113 of the intramedullary nail so that the leading end part 112 of the intramedullary nail is held along the first direction 111. The jig comprising a first guide 154 for drilling for the femoral neck screw, the first guide defining an aiming line 151 intersecting the first direction 111 at intersection 152. Preferably, the angle between the aiming line 151 and the first direction 111 is equal to the angle between the first direction 111 and the major axis 121 of the neck screw 120, see also Fig. 1. The first guide154 is positioned so that the intersection 152 between the aiming line 151 and the first direction 111 lies beyond the extension of the intramedullary nail 110, when the intramedullary nail is connected to the jig 150.

To see why the intersection 152 lies beyond the extension of the intramedullary nail 110 we return to Figs. 5A-C. In order to position the neck screw relative to the IM nail, the IM nail 110 is first inserted into the femur while attached to the jig 150, Fig 5A. Since the IM nail is to go through the through-hole of the neck screw, it cannot be inserted to its final position before the neck screw is in place. The insertion is therefore stopped when the aiming line points along the desired position of the major axis 121 of the femoral neck screw 120, and at this position, the IM nail shall not intersect the aiming line. Preferably the desired position of the femoral neck screw is when its major axis at least substantially overlaps with the extended line from the center of caput femoris and parallel to the collum axis in the AP plane.

The step of retrogradely inserting an intramedullary nail 110 in a femur comprises providing a jig 150 for inserting the intramedullary nail 110, the jig being configured to form a rigid mechanical connection to the trailing end part 113 of the intramedullary nail so that the leading end part 112 of the intramedullary nail is held along a first direction 111, the jig comprising a first guide 154 for drilling for the femoral neck screw, the first guide defining an aiming line 151 intersecting the first direction, wherein the intersection 152 between the aiming line and the first direction lies beyond the extension of the intramedullary nail, when the intramedullary nail is connected to the jig. The step further comprises inserting the IM nail using the jig until the aiming line at least substantially overlaps with the extended line from the center of caput femoris and parallel to the collum axis in the AP plane.

Intramedullary nails of different lengths will be used for different patients, and thus the position of the first guide on the jig may depend on the length of the nail. In one embodiment, the first guide may be fixed at a position where the intersection 152 lies beyond the extend of the longest IM nail produced. This has the advantage of simplifying the jig and the use of the system.

In another embodiment, the system further comprises a set of instructions comprising instructions for a user to use a combination of a guide and intramedullary nail length for which the intersection 152 between the aiming line and the first direction lies beyond the extension of the intramedullary nail, when the intramedullary nail is connected to the jig. A distance 153 from the intermedullary nail 110 to the intersection 152 should be larger than the cross-sectional radius of the neck screw at the position of the through-hole, please refer to Fig. 6. Thereby, the drill or the neck screw will not hit the IM nail upon insertion. At the same time, the longer the distance 153 from the intermedullary nail 110 to the intersection 152, the more difficult it may be to align the through-hole 125 with the leading end part 112 of the IM nail. This alignment involves adjusting the insertion depth of the neck screw 120 along its major axis 121 as well as the rotation of the neck screw around the major axis under fluoroscopy control. Since the insertion depth and the rotation are related via the lead of the thread 124 of the neck screw (see Fig. 2), some leeway in the fitting of the IM nail in the through-hole may be desired. If the tip of the IM nail is too far away, proper alignment becomes more difficult to estimate. In addition, if the distance 153 is unnecessary large, there will be an unnecessary large lever in the jig which influences the stiffness and thus the precision of the system negatively. Preferably, the distance 153 from the intermedullary nail 110 to the intersection 152 lies in the interval [1,5 cm; 8 cm], such as in the interval [1,5 cm; 4 cm].

In a further embodiment, also illustrated in Fig. 6, a proper combination of a guide and intramedullary nail length for the system is facilitated by the jig comprising at least a second guide for drilling for the femoral neck screw and defining an aiming line, a position of the second guide 155 being a translation of the position of the first guide 154 along a line at least substantially parallel to the first direction 111. Hence, different guides correspond to different lengths of the intramedullary nail. In order to assist the user in selecting the right guide, the system further comprises instructions for a user to, for a given a length of the intramedullary nail, use the one of the first and second guides (154, 155) that results in a distance 153 between the intersection 152 and the intramedullary nail 110 being closer to, but larger than, a radius of the femoral neck screw 120 at the position of the through-hole 125. The user need not try to estimate the distance 153 since it can be determined for each guide as a function of IM nail length at fabrication. The instructions may simply list the proper guide for a given length of IM nail, e.g. in the form of color codes or IM nail lengths written on the jig next to each guide.

In another embodiment of the system, a length of the jig 150 below a position of the first guide 154 can be adjusted so that the intersection 152 between the aiming line 151 and the first direction 111 can be moved along the first direction. Hence, the jig 150 can be adjusted to fit a range of IM nail lengths, providing the advantage that the distance 153 between the intersection and the IM nail can be adjusted continuously or in smaller increments than in the above embodiment comprising a second guide. The system may further comprise instructions for a user to, for a given a length of the intramedullary nail to be connected to the jig, adjust the length of the jig so that the distance 153 between the intersection and the intramedullary nail is as small as possible while being larger than a radius of the femoral neck screw at the through-hole. Again, the user need not try to estimate the distance 153 since it can be determined as a function of set jig length and IM nail length at fabrication. The instructions may simply list the proper jig length for a given length of IM nail, e.g. in the form of color codes or IM nail lengths written as a scale on the adjustable part of the jig.

Figure 7 illustrates a jig 150 for inserting an intramedullary nail 110 and aligning a femoral neck screw 120 relative to the intramedullary nail in osteosynthesis of femoral fractures according to the disclosure. The jig 150 has a J-shape with a short end part 157, a curved middle part 158, and a long end part 159, the short end part 157 being configured to form a rigid mechanical connection 156 to a trailing end part 113 of the intramedullary nail 110 so that the intramedullary nail is held along a first direction 111 at least substantially parallel to the long end part 159, the long end part comprising a guide 154 for drilling for the femoral neck screw, the guide defining an aiming line 151 intersecting the first direction 111. A length of the long end part 159 can be adjusted so that an intersection 152 between the aiming line and the first direction can be moved along the first direction. In a further embodiment, the jig is provided with instructions for a user to adjust the length of the long end part of the jig so that the intersection 152 lies beyond the extension of the intramedullary nail, when the intramedullary nail is connected to the jig.

In the following, a method for designing an IM nail according to the disclosure that is customized for a given patient is described.

Based on 3D-CT scan of the complete femur with an intertrochanteric fracture it is possible to design a nail that gives the maximal fit and strength of the nail. Opposite, it will also be possible to delineate those patients, who may not be candidates for this implant. For example, there has been a former fracture, which has healed in malalignment, there may be too much bone formation at the former fracture site, or there may be a total knee implant which may hinder osteosynthesis of the femur.

The retrograde femoral metallic nail will be made of Cobalt-28 Chromium-6Molybdenum alloys for surgical implants (Wrought) (UNS R31537, UNS R31538, and UNS R31539). - The maximal thickness of the nail can be measured. The load to failure of the implant can be estimated and plan for weight loading during rehabilitation can be estimated.

In cases with leg length discrepancy the implant may able to dynamize i.e. further shortening of the femoral bone should be expected and the dynamization should be adjusted to avoid the nail of re-entering the knee joint. The implant thickness will typically be at least 7-10 mm in thickness for humans.

The 3D-CT scan of the femoral anatomy will make it possible to produce a custom-made IM nail. The following parameters may be relevant to obtain the best IM nail fit together with best IM nail geometry: outer diameter and inner diameter of femur, centre of inner and outer surface of the IM nail, thickness of the IM nail, same thickness versus asymmetric thickness of the IM nail along the first direction.

The 3D-CT scanning of the femur hip will outline the bony parts of the pelvis/acetabulum and the femoral head. The digital data are downloaded in pixels and voxels with Hounsfield scale values and 3D geometric parameters can be determined based on digital 3D-programs such as Philips Intellispace Portal (ISP) or 3D Slicer 4.6, which is an open program from *harvard.edu,* only approved for research. The cortocal bony parts of the femur can be image segmented from the trabecular bony part. With two-dimensional CT-images the geometry of the space between the two bony parts can be determined. All the above-mentioned parameters will be validated to give the best fit and subsequently also make assessment of the possibility of an implant that can possess sufficient material strength (thickness). The CT-scanners have a detection limit of 0.5 mm regarding cartilage *(*Anderson et al, 2010 Radiology*)* and bone.

The free digital movement of the two segmented bony parts of the femur makes it possible to assess the fit of the implant. For routine use the 3D-CT may be converted, so the non-cortical bony part of the scanning can be visualized in 3D to construct the implant. The processing of the data may initially be "hand-made" based on slicing of the 3D image to obtain the separate bone structures in 3D.

The use of the terms "first", "second", "third" and "fourth", "primary", "secondary", "tertiary" etc. does not imply any particular order but are included to identify individual elements. Furthermore, the labelling of a first element does not imply the presence of a second element and vice versa. It is to be noted that the word "comprising" does not necessarily exclude the presence of other elements or steps than those listed. It is to be noted that the words "a" or "an" preceding an element do not exclude the presence of a plurality of such elements. It should further be noted that any reference signs do not limit the scope of the claims.

## Claims

1. An orthopaedic implant (100) for osteosynthesis of femoral fractures comprising:
an intramedullary nail (110) for retrograde insertion in the femur, the intramedullary nail comprising a leading end part (112) to be positioned proximally in the femur and a trailing end part (113) to be positioned distally in the femur;
a femoral neck screw (120) elongated along a major axis (121) and an at least partial, external thread (124) running along the major axis in a first end part (122) of the femoral neck screw for fastening the first end part subchondrally in a femoral head by a screwing action, the femoral neck screw comprising a through-hole (125) configured to receive the leading end part of the intramedullary nail along a first direction (111) having an angle between 110° and 150° to the major axis;
**characterized in that** the intramedullary nail comprises a stop (115) for engaging the femoral neck screw to prevent the leading end part from extending so far through the through-hole as to reach the cortex of the greater trochanter.

2. The orthopaedic implant according to claim 1, wherein the stop of the intramedullary nail involves a tapering with a smaller cross section nearer its leading end part and wherein the through-hole has a tapering with a larger cross section on a side for receiving the leading end part, the taperings being complementary to stop the intramedullary nail from extending more than 3 cm through the through-hole and laterally fixate the intramedullary nail relative to the femoral neck screw when the taperings engage.

3. The orthopaedic implant according to claim 1 or 2, wherein the leading end part of the intramedullary nail and the femoral neck screw involves structural features (116, 119, 126, 140) for preventing rotation of the intramedullary nail around the first direction and/or laterally fixating the intramedullary nail relative to the femoral neck screw after the leading end part has been received by the through-hole.

4. The orthopaedic implant according to any of the preceding claims, wherein the neck screw is cannulated along at least part of the major axis and comprises, in a second end part (123) opposite the first end part, an internal thread (117) along the cannulation (118), further comprising a set screw (140) having an external thread (141) corresponding to the internal thread along the cannulation and having a primary end part (142) adapted to engage the leading end part of the intramedullary nail when the leading end part is received by the through-hole and the set screw is screwed towards the first end part of the femoral neck screw.

5. The orthopaedic implant according to claim 4, wherein the leading end part comprises a recess (119) or a hole for receiving the primary end part of the set screw to prevent rotation of the intramedullary nail around the first direction when the leading end part is received by the through-hole and the set screw is screwed towards the first end part of the femoral neck screw.

6. The orthopaedic implant according to any of the preceding claims, wherein the stop of the intramedullary nail is positioned to allow the leading end part to extend at least 3 mm beyond a proximal rim (134) of the through-hole and prevent the leading end part from extending more than 3 centimeters through the through-hole when the stop engages the femoral neck screw.

7. The orthopaedic implant according to any of the preceding claims, wherein the leading end part of the intramedullary nail is configured to engage a proximal rim (134) of the through-hole when the stop engages the femoral neck screw

8. A system (101) for osteosynthesis of femoral fractures comprising:
an orthopaedic implant (100) according to any of claims 1-7; and
a jig (150) for inserting the intramedullary nail and aligning the femoral neck screw relative to the intramedullary nail, the jig being configured to form a rigid mechanical connection to the trailing end part of the intramedullary nail so that the leading end part of the intramedullary nail is held along the first direction, the jig comprising a first guide (154) for drilling for the femoral neck screw, the first guide defining a first aiming line (151) intersecting the first direction;
wherein an intersection (152) between the first aiming line and the first direction lies beyond the extension of the intramedullary nail, when the intramedullary nail is connected to the jig.

9. The system according to claim 8, further comprising a set of instructions comprising instructions for a user to use a combination of a guide and intramedullary nail length for which the intersection between the first aiming line and the first direction lies beyond the extension of the intramedullary nail, when the intramedullary nail is connected to the jig.

10. The system according to any of claims 8-9, wherein the jig comprises at least a second guide (155) for drilling for the femoral neck screw and defining a second aiming line, a position of the second guide being a translation of the position of the first guide along a line at least substantially parallel to the first direction, the system further comprising a set of instructions comprising instructions for a user to, for a given a length of the intramedullary nail to be connected to the jig, use the one of the first and second guides that results in a distance between the intersection and the intramedullary nail being closer to, but larger than, a radius of the femoral neck screw at the through-hole.

11. The system according to any of claims 8-9, wherein a length of the jig below a position of the first guide can be adjusted so that the intersection between the first aiming line and the first direction can be moved along the first direction, further comprising a set of instructions comprising instructions for a user to, for a given a length of the intramedullary nail to be connected to the jig, adjust the length of the jig so that a distance between the intersection and the intramedullary nail is as small as possible while being larger than a radius of the femoral neck screw at the through-hole.

12. The system according to any of claims 8-11, wherein the jig has a J-shape with a short end part (1567), a curved middle part (158), and a long end part (159), wherein the long end part comprises a scale indicating the length to which the long end part should be adjusted for an intramedullary nail of a given length held at the short end part.

13. The system according to claim 12, wherein the scale is calibrated so that the intersection between the first aiming line and the first direction lies beyond the extension of an intramedullary nail with the given length, when the intramedullary nail is held at the short end part.

## Patentansprüche

1. Orthopädisches Implantat (100) zur Osteosynthese von Femurfrakturen, Folgendes umfassend:
einen Marknagel (110) zum retrograden Einsetzen in das Femur, wobei der Marknagel einen Vorderendteil (112) zum proximalen Positionieren in dem Femur und einen Hinterendteil (113) zum distalen Positionieren in dem Femur umfasst;
eine Schenkelhalsschraube (120), die entlang einer Hauptachse (121) länglich ausgebildet ist, und ein mindestens teilweises Außengewinde (124), das entlang der Hauptachse in einem ersten Endteil (122) der Schenkelhalsschraube verläuft, um den ersten Endteil durch eine Schraubbewegung subchondral in einem Femurkopf zu befestigen, wobei die Schenkelhalsschraube ein Durchgangsloch (125) umfasst, das dazu konfiguriert ist, den Vorderendteil des Marknagels entlang einer ersten Richtung (111) aufzunehmen, die einen Winkel zwischen 110° und 150° zu der Hauptachse aufweist;
**dadurch gekennzeichnet, dass** der Marknagel einen Anschlag (115) zum Eingreifen in die Schenkelhalsschraube umfasst, um zu verhindern, dass sich der Vorderendteil so weit durch das Durchgangsloch erstreckt, dass er die Kortikalis des Trochanter major erreicht.

2. Orthopädisches Implantat nach Anspruch 1, wobei der Anschlag des Marknagels eine Verjüngung mit einem kleineren Querschnitt nahe seinem Vorderendteil aufweist und wobei das Durchgangsloch eine Verjüngung mit einem größeren Querschnitt auf einer Seite zum Aufnehmen des Vorderendteils aufweist, wobei die Verjüngungen komplementär sind, um zu verhindern, dass sich der Marknagel mehr als 3 cm durch das Durchgangsloch erstreckt, und um den Marknagel seitlich relativ zu der Schenkelhalsschraube zu fixieren, wenn die Verjüngungen ineinandergreifen.

3. Orthopädisches Implantat nach Anspruch 1 oder 2, wobei der Vorderendteil des Marknagels und der Schenkelhalsschraube Strukturmerkmale (116, 119, 126, 140) aufweist, um eine Drehung des Marknagels um die erste Richtung zu verhindern und/oder den Marknagel seitlich relativ zu der Schenkelhalsschraube zu fixieren, nachdem der Vorderendteil durch das Durchgangsloch aufgenommen wurde.

4. Orthopädisches Implantat nach einem der vorhergehenden Ansprüche, wobei die Halsschraube entlang mindestens eines Teils der Hauptachse kanüliert ist und in einem dem ersten Endteil gegenüberliegenden zweiten Endteil (123) ein Innengewinde (117) entlang der Kanülierung (118) umfasst und ferner eine Stellschraube (140) mit einem Außengewinde (141), das dem Innengewinde entlang der Kanülierung entspricht, und mit einen primären Endteil (142), der zum Eingriff mit dem Vorderendteil des Marknagels angepasst ist, wenn der Vorderendteil durch das Durchgangsloch aufgenommen ist und die Stellschraube in Richtung des ersten Endteils der Schenkelhalsschraube geschraubt wird, umfasst.

5. Orthopädisches Implantat nach Anspruch 4, wobei der Vorderendteil eine Aussparung (119) oder ein Loch zum Aufnehmen des primären Endteils der Stellschraube umfasst, um eine Drehung des Marknagels um die erste Richtung zu verhindern, wenn der Vorderendteil durch das Durchgangsloch aufgenommen ist und die Stellschraube in Richtung des ersten Endteils der Schenkelhalsschraube geschraubt wird.

6. Orthopädisches Implantat nach einem der vorhergehenden Ansprüche, wobei der Anschlag des Marknagels so positioniert ist, dass sich der Vorderendteil mindestens 3 mm über einen proximalen Rand (134) des Durchgangslochs hinaus erstrecken kann und dass sich der Vorderendteil nicht weiter als 3 Zentimeter durch das Durchgangsloch erstrecken kann, wenn der Anschlag in die Schenkelhalsschraube eingreift.

7. Orthopädisches Implantat nach einem der vorhergehenden Ansprüche, wobei der Vorderendteil des Marknagels dazu konfiguriert ist, in einen proximalen Rand (134) des Durchgangslochs einzugreifen, wenn der Anschlag in die Schenkelhalsschraube eingreift.

8. System (101) zur Osteosynthese von Femurfrakturen, Folgendes umfassend:
ein orthopädisches Implantat (100) nach einem der Ansprüche 1-7; und
eine Spannvorrichtung (150) zum Einsetzen des Marknagels und Ausrichten der Schenkelhalsschraube relativ zu dem Marknagel, wobei die Spannvorrichtung dazu konfiguriert ist, eine starre mechanische Verbindung mit dem Hinterendteil des Marknagels zu bilden, sodass der Vorderendteil des Marknagels entlang der ersten Richtung gehalten wird, wobei die Spannvorrichtung eine erste Führung (154) zum Bohren für die Schenkelhalsschraube umfasst, wobei die erste Führung eine erste Ziellinie (151) definiert, die die erste Richtung schneidet;
wobei ein Schnittpunkt (152) zwischen der ersten Ziellinie und der ersten Richtung jenseits der Verlängerung des Marknagels liegt, wenn der Marknagel mit der Spannvorrichtung verbunden ist.

9. System nach Anspruch 8, ferner einen Satz von Anweisungen umfassend, die Anweisungen für einen Benutzer zur Verwendung einer Kombination aus einer Führung und einer Marknagellänge umfassen, bei der der Schnittpunkt zwischen der ersten Ziellinie und der ersten Richtung jenseits der Verlängerung des Marknagels liegt, wenn der Marknagel mit der Spannvorrichtung verbunden ist.

10. System nach einem der Ansprüche 8-9, wobei die Spannvorrichtung mindestens eine zweite Führung (155) zum Bohren für die Schenkelhalsschraube und zum Definieren einer zweiten Ziellinie umfasst, wobei eine Position der zweiten Führung eine Verschiebung der Position der ersten Führung entlang einer Linie ist, die mindestens im Wesentlichen parallel zu der ersten Richtung ist, wobei das System ferner einen Satz von Anweisungen umfasst, die Anweisungen für einen Benutzer umfassen, um für eine vorgegebene Länge des mit der Spannvorrichtung zu verbindenden Marknagels diejenige von der ersten und der zweiten Führung zu verwenden, die einen Abstand zwischen dem Schnittpunkt und dem Marknagel ergibt, der näher an einem Radius der Schenkelhalsschraube an dem Durchgangsloch liegt, aber größer als dieser ist.

11. System nach einem der Ansprüche 8-9, wobei eine Länge der Spannvorrichtung unterhalb einer Position der ersten Führung so eingestellt werden kann, dass der Schnittpunkt zwischen der ersten Ziellinie und der ersten Richtung entlang der ersten Richtung bewegt werden kann, ferner einen Satz von Anweisungen umfassend, die Anweisungen für einen Benutzer umfassen, um bei einer vorgegebenen Länge des mit der Spannvorrichtung zu verbindenden Marknagels die Länge der Spannvorrichtung so einzustellen, dass ein Abstand zwischen dem Schnittpunkt und dem Marknagel so klein wie möglich ist und gleichzeitig größer als ein Radius der Schenkelhalsschraube an dem Durchgangsloch ist.

12. System nach einem der Ansprüche 8-11, wobei die Spannvorrichtung eine J-Form mit einem kurzen Endteil (1567), einem gekrümmten Mittelteil (158) und einem langen Endteil (159) aufweist, wobei der lange Endteil eine Skala umfasst, die die Länge angibt, auf die der lange Endteil für einen an dem kurzen Endteil gehaltenen Marknagel einer vorgegebenen Länge eingestellt werden soll.

13. System nach Anspruch 12, wobei die Skala so kalibriert ist, dass der Schnittpunkt zwischen der ersten Ziellinie und der ersten Richtung jenseits der Verlängerung eines Marknagels mit der vorgegebenen Länge liegt, wenn der Marknagel an dem kurzen Endteil gehalten wird.

## Revendications

1. Implant orthopédique (100) pour l'ostéosynthèse de fractures fémorales comprenant :
un clou intramédullaire (110) pour une insertion rétrograde dans le fémur, le clou intramédullaire comprenant une partie d'extrémité avant (112) à positionner de manière proximale dans le fémur et une partie d'extrémité arrière (113) à positionner de manière distale dans le fémur ;
une vis de col de fémur (120) allongée le long d'un axe majeur (121) et un filetage externe au moins partiel (124) s'étendant le long de l'axe majeur dans une première partie d'extrémité (122) de la vis de col de fémur pour la fixation sous-chondrale de la première partie d'extrémité dans une tête fémorale par une action de vissage, la vis de col de fémur comprenant un trou traversant (125) configuré pour recevoir la partie d'extrémité avant du clou intramédullaire le long d'une première direction (111) ayant un angle entre 110° et 150° pour l'axe majeur ;
**caractérisé en ce que** le clou intramédullaire comprend une butée (115) pour venir en prise avec la vis de col de fémur pour empêcher la partie d'extrémité avant de s'étendre si loin à travers le trou traversant qu'elle atteint le cortex du grand trochanter.

2. Implant orthopédique selon la revendication 1, dans lequel la butée du clou intramédullaire comporte un effilement avec une section transversale plus petite plus près de sa partie d'extrémité avant et dans lequel le trou traversant a un effilement avec une section transversale plus grande sur un côté pour recevoir la partie d'extrémité avant, les effilements étant complémentaires pour empêcher le clou intramédullaire de s'étendre sur plus de 3 cm à travers le trou traversant et fixer latéralement le clou intramédullaire par rapport à la vis de col de fémur lorsque les effilements viennent en prise.

3. Implant orthopédique selon la revendication 1 ou 2, dans lequel la partie d'extrémité avant du clou intramédullaire et la vis de col de fémur comportent des éléments structurels (116, 119, 126, 140) pour empêcher la rotation du clou intramédullaire autour de la première direction et/ou fixer latéralement le clou intramédullaire par rapport à la vis de col de fémur après que la partie d'extrémité avant a été reçue par le trou traversant.

4. Implant orthopédique selon l'une quelconque des revendications précédentes, dans lequel la vis de col est canulée le long d'au moins une partie de l'axe majeur et comprend, dans une seconde partie d'extrémité (123) opposée à la première partie d'extrémité, un filetage interne (117) le long de la canulation (118), comprenant en outre une vis de réglage (140) ayant un filetage externe (141) correspondant au filetage interne le long de la canulation et ayant une partie d'extrémité principale (142) conçue pour venir en prise avec la partie d'extrémité avant du clou intramédullaire lorsque la partie d'extrémité avant est reçue par le trou traversant et que la vis de réglage est vissée vers la première partie d'extrémité de la vis de col de fémur.

5. Implant orthopédique selon la revendication 4, dans lequel la partie d'extrémité avant comprend un évidement (119) ou un trou pour recevoir la partie d'extrémité principale de la vis de réglage pour empêcher la rotation du clou intramédullaire autour de la première direction lorsque la partie d'extrémité avant est reçue par le trou traversant et que la vis de réglage est vissée vers la première partie d'extrémité de la vis de col de fémur.

6. Implant orthopédique selon l'une quelconque des revendications précédentes, dans lequel la butée du clou intramédullaire est positionnée pour permettre à la partie d'extrémité avant de s'étendre d'au moins 3 mm au-delà d'un rebord proximal (134) du trou traversant et empêcher la partie d'extrémité avant de s'étendre sur plus de 3 centimètres à travers le trou traversant lorsque la butée vient en prise avec la vis de col de fémur.

7. Implant orthopédique selon l'une quelconque des revendications précédentes, dans lequel la partie d'extrémité avant du clou intramédullaire est configurée pour venir en prise avec un rebord proximal (134) du trou traversant lorsque la butée vient en prise avec la vis de col de fémur.

8. Système (101) pour l'ostéosynthèse de fractures fémorales comprenant :
un implant orthopédique (100) selon l'une quelconque des revendications 1 à 7 ; et
un gabarit (150) pour insérer le clou intramédullaire et aligner la vis de col de fémur par rapport au clou intramédullaire, le gabarit étant configuré pour former une liaison mécanique rigide avec la partie d'extrémité arrière du clou intramédullaire de sorte que la partie d'extrémité avant du clou intramédullaire soit maintenue le long de la première direction, le gabarit comprenant un premier guide (154) pour le perçage de la vis de col de fémur, le premier guide définissant une première ligne de visée (151) coupant la première direction ;
dans lequel une intersection (152) entre la première ligne de visée et la première direction se situe au-delà de l'extension du clou intramédullaire, lorsque le clou intramédullaire est relié au gabarit.

9. Système selon la revendication 8, comprenant en outre un ensemble d'instructions comprenant des instructions permettant à un utilisateur d'utiliser une combinaison d'un guide et d'une longueur de clou intramédullaire pour laquelle l'intersection entre la première ligne de visée et la première direction se situe au-delà de l'extension du clou intramédullaire, lorsque le clou intramédullaire est relié au gabarit.

10. Système selon l'une quelconque des revendications 8 et 9, dans lequel le gabarit comprend au moins un second guide (155) pour le perçage de la vis de col de fémur et la définition d'une seconde ligne de visée, une position du second guide étant une translation de la position du premier guide le long d'une ligne au moins sensiblement parallèle à la première direction, le système comprenant en outre un ensemble d'instructions comprenant des instructions permettant à un utilisateur, pour une longueur donnée du clou intramédullaire à relier au gabarit, d'utiliser celui des premier et second guides entraînant une distance entre l'intersection et le clou intramédullaire plus proche, mais plus grande, d'un rayon de la vis de col de fémur au niveau du trou traversant.

11. Système selon l'une quelconque des revendications 8 et 9, dans lequel une longueur du gabarit inférieure à une position du premier guide peut être réglée de sorte que l'intersection entre la première ligne de visée et la première direction puisse être déplacée le long de la première direction, comprenant en outre un ensemble d'instructions comprenant des instructions permettant à un utilisateur, pour une longueur donnée du clou intramédullaire à relier au gabarit, de régler la longueur du gabarit de sorte qu'une distance entre l'intersection et le clou intramédullaire soit aussi petite que possible tout en étant plus grande qu'un rayon de la vis de col de fémur au niveau du trou traversant.

12. Système selon l'une quelconque des revendications 8 à 11, dans lequel le gabarit a une forme en J avec une partie d'extrémité courte (1567), une partie médiane incurvée (158) et une partie d'extrémité longue (159), dans lequel la partie d'extrémité longue comprend une échelle indiquant la longueur à laquelle la partie d'extrémité longue doit être réglée pour un clou intramédullaire d'une longueur donnée maintenu au niveau de la partie d'extrémité courte.

13. Système selon la revendication 12, dans lequel l'échelle est calibrée de sorte que l'intersection entre la première ligne de visée et la première direction se situe au-delà de l'extension d'un clou intramédullaire de longueur donnée, lorsque le clou intramédullaire est maintenu au niveau de la partie d'extrémité courte.
